# DEMANDE DE BREVET EUROPEEN

(11) **EP 1 652 509 A2**
(43) Date de publication de la demande: **03.05.2006**
(21) Numéro de dépôt: 05292222.6
(22) Date de dépôt: 21.10.2005
(51) Int. Cl.: A61K 8/18

(54) **Composition capillaire épaissie comprenant un polyuréthane de haut poids moléculaire**

(30) Priorité: 28.10.2004 FR 0452468
(71) Demandeur: L'OREAL, 75008 Paris (FR)
(72) Inventeur: Rollat-Corvol, Isabelle, 75017 Paris (FR); Cothias, Pascale, 78180 Montigny le Bretonneux (FR)
(74) Mandataire: Bourdeau, Françoise

(57) **Abrégé**

L'invention a pour objet une composition cosmétique capillaire, notamment un gel, comprenant au moins un polymère polyuréthane de haut poids moléculaire et au moins un épaississant. L'invention vise également un procédé cosmétique capillaire comprenant l'application de cette composition sur les cheveux ainsi que son utilisation pour fixer et/ou maintenir la coiffure.

## Description

L'invention a pour objet une composition cosmétique capillaire, notamment un gel, comprenant au moins un polymère polyuréthane de haut poids moléculaire et au moins un épaississant. L'invention vise également un procédé cosmétique capillaire comprenant l'application de cette composition sur les cheveux ainsi que son utilisation pour fixer et/ou maintenir la coiffure.

Au sens de la présente invention, on entend par « polyuréthane de haut poids moléculaire », un polyuréthane de poids moléculaire moyen en nombre compris entre 400 000 et 5 000 000, bornes incluses.

Au sens de la présente invention, on entend par *« gel »*, une composition initialement liquide, épaissie grâce à un agent ayant des propriétés épaississantes et/ou gélifiantes, ayant in fine une viscosité minimale de 160 cps, de préférence 250 cps à 25°C (viscosimètre Rhéomat 180 - mobile 2 - lecture après 30s).

On entend par « *épaississant* », tout composé, capable d'augmenter la viscosité d'une composition cosmétique.

Il est connu des produits de fixation et/ou de maintien de la coiffure se présentant sous diverses formes, notamment sous forme de gels. Si ceux-ci permettent souvent la fixation durable de la coiffure, ils présentent néanmoins divers inconvénients.

En particulier, les polymères couramment utilisés en tant qu'agents fixants dans les gels ne permettent pas de conserver la mise en forme de la chevelure lorsque celle-ci est en contact avec de l'eau liquide pendant une durée prolongée, telle que par exemple une mise en contact avec la pluie, la transpiration ou lors de bains de mer ou de bains en piscine.

Il est également reproché aux gels de conduire à une coiffure résistant mal à l'humidité de l'air.

Pour résoudre les problèmes ci-dessus, il a déjà été proposé de réaliser des produits cosmétiques capillaires comprenant un polymère fixant et un agent épaississant avec des additifs éventuels. Néanmoins, les compositions connues à ce jour comprenant ces constituants ne donnent pas encore totalement satisfaction.

Le problème posé par l'invention est de fournir une composition cosmétique capillaire, notamment sous forme de gel, qui soit améliorée par rapport aux compositions de l'art antérieur.

De manière surprenante et inattendue, la Demanderesse a découvert qu'en sélectionnant judicieusement le polymère fixant, il est possible de résoudre les problèmes posés ci-dessus.

L'invention a pour objet une composition cosmétique se présentant sous la forme d'un gel et comprenant, dans un milieu cosmétiquement acceptable, au moins un polyuréthane de poids moléculaire moyen en nombre compris entre 400 000 et 5 000 000 g/mol et au moins un épaississant.

Ces compositions permettent de fixer et de mettre en forme les coiffures et aussi de conserver la mise en forme des coiffures, lorsque celles-ci sont au contact de l'eau liquide, pendant une durée prolongée. Elles permettent aussi d'obtenir une coiffure résistant l'humidité de l'air.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition dans une formulation cosmétique capillaire, destinée notamment au maintien et/ou à la mise en forme de la coiffure.

Par « durée prolongée » au sens de la présente demande, on entend une mise en contact avec l'eau d'une durée minimale d'une minute, de préférence 10 minutes et de manière encore plus préférée 20 minutes.

Selon l'invention, le « polyuréthane de haut poids moléculaire » et « l'épaississant » sont des composés distincts l'un de l'autre.

### POLYURETHANES DE HAUT POIDS MOLECULAIRE

Par polyuréthane, on entend au sens de la présente invention, des polycondensats comprenant au moins une séquence polyuréthane. Ils sont décrits en particulier dans les brevets EP 0 751 162, EP 0 637 600, FR 2 743 297 et EP 0 648 485 dont la Demanderesse est titulaire, ainsi que les brevets EP 0 656 021 ou WO 94/03510 de la Société BASF et EP 0 619 111 de la Société National Starch.

Les polyuréthanes utilisés conformément à l'invention peuvent être solubles dans le milieu aqueux cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polyuréthane non associatif.

Selon l'invention, on peut utiliser tout type de tensioactif dans ladite dispersion, mais de préférence un tensioactif non ionique. La taille moyenne des particules du polyuréthane non associatif dans la dispersion est de préférence comprise entre 0,1 et 1 micromètre.

A titre d'exemple, le polyuréthane peut être formé par un arrangement de blocs, cet arrangement étant obtenu notamment à partir de :
(1) au moins un composé qui contient deux ou plus de deux atomes d'hydrogène actifs par molécule ;
(2) au moins un diol ou un mélange de diols contenant des fonctions acides ou leurs sels ; et
(3) au moins un di- ou polyisocyanate.

Avantageusement, les composés (1) sont choisis dans le groupe comprenant les diols, les diamines, les polyesterols, les polyétherols ou leurs mélanges.

Les composés (1) qui sont préférés sont les polyéthylèneglycols et les polypropylèneglycols linéaires, en particulier ceux qui sont obtenus par réaction de l'oxyde d'éthylène ou de propylène avec l'eau ou du diéthylène ou du dipropylèneglycol en présence d'hydroxyde de sodium en tant que catalyseur. Ces polyalkylèneglycols ont généralement une masse moléculaire comprise entre environ 600 et 20 000.

D'autres composés organiques préférés sont ceux qui ont des groupes mercapto, amino, carboxyle ou hydroxyle. Parmi ceux-ci, on cite plus particulièrement les composés polyhydroxylés tels que les polyéther-diols, les polyester-diols, les polyacétal-diols, les polyamide-diols, les polyester-polyamide-diols, les poly(alkylène éther)-diols, les polythioéther-diols et les polycarbonatediols.

Les polyéther-diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéther-diols appropriés sont par exemple préparés par condensation d'oxydes d'alkylène et d'alcools polyhydriques, tels que l'éthylèneglycol, le 1,2-propylèneglycol et le 1,4-butanediol.

Les polyester-diols, polyester-amides, polyamide-diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylèneglycol, le 1,2-propylèneglycol, le 1,4-butanediol, le néopentylglycol et l'hexanediol. On peut aussi utiliser des aminoalcools, par exemple l'éthanolamine. Des diamines appropriées pour préparer les amide-polyesters sont l'éthylène-diamine et l'hexaméthylène-diamine.

Des polyacétals appropriés peuvent être préparés, par exemple, à partir de 1,4-butanediol ou d'hexanediol et de formaldéhyde. Des polythioéthers appropriés peuvent être préparés, par exemple, par réaction de condensation entre des thioglycols seuls ou en combinaison avec d'autres glycols tels que l'éthylèneglycol, le 1,2-propylèneglycol ou avec d'autres composés polyhydroxylés. Les composés polyhydroxylés contenant déjà des groupements uréthannes, des polyols naturels, qui peuvent être davantage modifiés, par exemple, l'huile de ricin et les carbohydrates peuvent également être utilisés.

Plus préférentiellement, le composé du groupe (1) est un polyestérol, notamment un polyester-diol formé par la réaction d'au moins un (di)-polyol (1ₐ) et d'au moins un acide (1_{b}). Le (di)-polyol (1ₐ) est en particulier choisi dans le groupe comprenant le néopentylglycol, le 1,4-butanediol, l'hexanediol, l'éthylèneglycol, le diéthylèneglycol, le propylèneglycol, le butylèneglycol, le néopentylglycol et (di)-polyéthylèneglycol. L'acide (1_{b}) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol-propanoïque (DMPA) ou un acide 2,2-hydroxyméthyl-carboxylique. En général, le composé (2) est utile en tant que bloc de couplage. En tant que composés (2), on préfère ceux comprenant au moins un poly(acide carboxylique α,α-dihydroxylé).

Les composés (2) particulièrement préférés conformément à l'invention sont ceux choisis dans le groupe comprenant l'acide 2,2-di-(hydroxyméthyl)acétique, l'acide 2,2-dihydroxyméthylpropionique, l'acide 2,2-dihydroxyméthylbutyrique, l'acide 2,2-dihydroxyméthylpentanoïque.

Le di- ou polyisocyanate (3) peut être choisi en particulier dans le groupe comprenant l'hexaméthylène-diisocyanate, l'isophorone-diisocyanate (IDPI), le toluylène-diisocyanate, le diphénylméthane-4,4'-diisocyanate (DPMD) et le dicyclohexylméthane-4,4'-diisocyanate (DCMD), le méthylène-di-p-phényl-diisocyanate, le méthylène-bis(4-cyclohexylisocyanate), les toluène-diisocyanates, le 1,5-naphtalène-diisocyanate, le 4,4'-diphénylméthane-diisocyanate, le 2,2'-diméthyl-4,4'-diphénylméthane-diisocyanate, le 1,3-phénylène-diisocyanate, le 1,4-phénylène diisocyanate, des mélanges de 2,4- et de 2,6-toluène-diisocyanates, le 2,2'-dichloro-4,4'-diisocyanato-diphénylméthane, le 2,4-dibromo-1,5-diisocyanato naphtalène, le butane-1,4-diisocyanate, l'hexane-1,6-diisocyanate, et le cyclohexane-1,4-diisocyanate.

Le polyuréthane non associatif peut être formé à l'aide d'un composé supplémentaire (4) servant en général à allonger sa chaîne. Ces composés (4) peuvent être choisis dans le groupe comprenant notamment les glycols saturés ou insaturés tels que l'éthylèneglycol, le diéthylèneglycol, le néopentylglycol, le triéthylène glycol ; les aminoalcools tels que l'éthanolamine, la propanolamine, la butanolamine ; les amines primaires hétérocycliques, aromatiques, cycloaliphatiques, et aliphatiques ; les diamines ; les acides carboxyliques tels que les acides carboxyliques aliphatiques, aromatiques et hétérocycliques comme les acides oxalique, succinique, glutarique, adipique, sébacique et téréphtalique ; et les acides aminocarboxyliques. Les composés (4) préférés sont les diols aliphatiques.

Les polyuréthanes utilisés selon l'invention peuvent également être encore formés à partir de composés supplémentaires (5) ayant un squelette siliconé tels que les polysiloxanes, les polyalkylsiloxanes ou les polyarylsiloxanes, notamment les polyéthylsiloxanes, les polyméthylsiloxanes et les polyphénylsiloxanes, comportant éventuellement des chaînes hydrocarbonées greffées sur les atomes de silicium.

Les polyuréthanes utilisés avantageusement comprennent un motif répétitif de base répondant à la formule générale (I) :

-O-B-O-CO-NH-R-NH-CO- (I)

dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes hydrocarbonés aliphatiques en C₁ à C₂₀, cycloaliphatiques en C₃ à C₂₀ et aromatiques en C₆ à C₂₀, comme par exemple les groupes alkylène en C₁ à C₂₀, arylène en C₆ à C₂₀, cycloalkylène en C₃ à C₂₀, ou leurs combinaisons, ces groupes étant substitués ou non.

Le groupe R est avantageusement choisi parmi les groupes répondant aux formules suivantes :

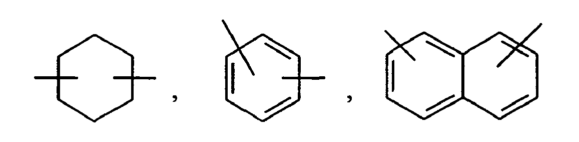

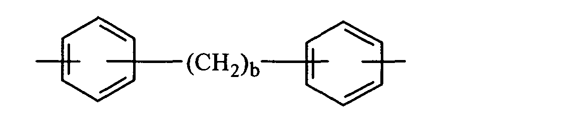

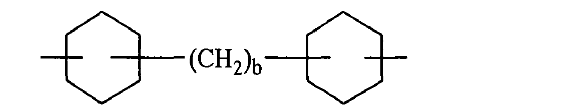

― (CH₂)_{c}―

dans lesquelles b est un nombre entier compris entre 0 et 3, et c un nombre entier compris entre 1 et 20, de préférence compris entre 2 et 12.

En particulier, le groupe R est choisi parmi les groupes hexaméthylène, 4,4'-biphénylèneméthane, 2,4- et/ou 2,6-tolylène, 1,5-naphtylène, p-phénylène, méthylène- 4,4-bis-cyclohexyle et le groupe divalent dérivé de l'isophorone.

Le polyuréthane utilisé dans la présente invention peut comprendre en outre au moins une séquence polysiloxane.

Les polyuréthanes de l'invention sont, de préférence, non associatifs, c'est-à-dire qu'ils ne contiennent pas dans leur structure de chaîne alkyle ou alcényle comportant plus de 10 atomes de carbone.

Les polyuréthanes sont notamment présents dans la composition selon l'invention, en une quantité de 0,05 à 40 % en poids, de préférence de 0,1 à 20 % en poids, et mieux encore de 1 à 8 % en poids par rapport au poids total de la composition de traitement cosmétique des matières kératiniques.

Avantageusement, le polyuréthane présente une masse moléculaire comprise entre environ 400 000 et 3 000 000, de préférence entre 1 000 000 et 2 500 000.

Plus avantageusement, le polyuréthane est l'Avalure UR-450 commercialisé par la Société Novéon, lequel est un copolymère anionique formé de PPG-17 (polypropylène glycol de nombre de motifs n=17)/IPDI (isophorone diisocyanate)/DMPA (diméthylol propionic acid). En milieu aqueux, il se présente sous la forme d'une dispersion. Son poids moléculaire est 1 830 000 g/mol.

### EPAISSISSANTS

La composition comprend avantageusement, en pourcentage relatif en poids de la composition, de 0,01 à 20 % d'épaississant, et de préférence, de 0,05 à 10 %.

De préférence, l'agent épaississant est choisi parmi les polymères naturels ou naturels modifiés (i), les copolymères réticulés d'acide acrylique et/ou méthacrylique (ii), les polyacrylamides épaississants réticulés (iii) et les polymères associatifs comportant au moins un motif hydrophile et au moins une chaîne grasse (iv) .

Par « épaississant naturel modifié » (i), on entend selon l'invention tout polymère épaississant obtenu par modification chimique simple à partir du polymère naturel lui-même.

On peut citer comme épaississant naturels ou naturels modifiés (i) convenant pour l'invention les gommes cellulose, de scléroglucane, de gellane, de rhamsan, les alginates, la gomme de karaya, la farine de caroube, les gommes de guar modifiées ou non et leurs dérivés, comme hydroxypropylguar. On utilise notamment la gomme de guar et ses dérivés (hydroxypropylguar), les gommes d'origine naturelle microbienne (gomme de xanthane, gomme de scléroglucane).

Les copolymères d'acide acrylique et/ou méthacrylique réticulés (ii) sont notamment les copolymères acide acrylique/acrylate d'éthyle et les polymères carboxyvinyliques. De tels polymères sont notamment les "carbomer" (CTFA) vendus par la société GOODRICH sous la dénomination Carbopol.

Les polyacrylamides épaississants réticulés (iii) peuvent plus particulièrement être choisis parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium réticulés,
- les copolymères d'acrylamide et d'acide 2-acrylamido 2-méthyl propanesulfonique partiellement ou totalement neutralisés réticulés.

Comme copolymères réticulés d'acrylamide / acrylate d'ammonium, utilisés conformément à la présente invention, on peut plus particulièrement citer les copolymères acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce type de copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de 10⁻⁴ à 4.10⁻⁴ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau. Une telle émulsion est commercialisée sous le nom de "SEPIGEL 305" par la société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

Les copolymères non réticulés de méthacrylamide et de chlorure de méthacryloyloxyéthyl triméthylammonium sont par exemple les produits vendus sous les dénominations commerciales ROHAGIT KF 400 et KF720 par la société ROHM et Haas.

Les polymères associatifs (iv) de l'invention peuvent être non ioniques, cationiques, anioniques ou amphotères. Ils peuvent, par exemple, appartenir à la classe des polyuréthannes.

Les polyuréthannes associatifs sont des copolymères séquencés comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de C₆ à C₃₀ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthanne entre les séquences hydrophiles, d'où l'origine du nom.

Par extension figurent aussi parmi les polyuréthannes associatifs, des polymères dont les séquence hydrophiles sont liées par d'autres liaisons chimiques aux séquences lipophiles.

A titre d'exemple, des polymères associatifs utilisables dans l'invention, on peut citer le polymère C₁₆-OE₁₂₀-C₁₆ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en C₂₀ et à liaison uréthanne, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010 et le Sérad 1035 vendus par la société HULS, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS.

Les polymères utilisables dans l'invention sont en particulier ceux décrits dans l'article de G. Fonnum, J. Bakke et Fk. Hansen - Colloid Polym. Sci 271, 380.389 (1993).

Les polyuréthannes associatifs (iv) peuvent particulièrement être choisis parmi :
- les polyéthers polyuréthanes (a) susceptibles d'être obtenus par polycondensation d'au moins trois composés comprenant (i) au moins un polyéthylèneglycol comprenant de 50 à 500 moles d'oxyde d'éthylène, (ii) au moins un alcool gras en C₈-C₃₀ et (iii) au moins un diisocyanate et,
- les polyéthers polyuréthanes (b) susceptible d'être obtenus par polycondensation d'au moins trois composés comprenant (i) au moins un polyéthylèneglycol comprenant de 50 à 500 moles d'oxyde d'éthylène, (ii) au moins un alcool gras en C₈-C₃₀ différant de celui du polyéther polyuréthane (a) et (iii) au moins un diisocyanate.

De façon préférentielle, on utilisera des polyéthers polyuréthanes (a) et (b) pour lesquels le polyéthylène glycol comporte 150 ou 180 moles d'oxyde d'éthylène.

Toujours de façon préférentielle, on utilisera des polyéthers polyuréthanes (a) et (b) pour lesquels le diisocyanate est le méthylène bis(4-cyclohexylisocyanate).

Une composition plus particulièrement préférée selon l'invention est une composition telle que définie ci-dessus, pour laquelle, le polyéther polyuréthane (a) est obtenu par polycondensation d'au moins trois composés comprenant, un polyéthylèneglycol à 150 ou 180 moles d'oxyde d'éthylène, l'alcool stéarylique (C18) et le méthylène bis(4-cyclohexylisocyanate), et le polyéther polyuréthane (b) est obtenu par polycondensation d'au moins trois composés comprenant, un polyéthylèneglycol à 150 ou 180 moles d'oxyde d'éthylène, l'alcool décylique (C10) et le méthylène bis(4-cyclohexylisocyanate).

Parmi les polyéthers polyuréthanes (a), on peut citer le produit vendu par la société ROHM & HAAS sous la dénomination commerciale :
ACULYN 46, qui est un polycondensat comprenant au moins comme éléments, un polyéthylèneglycol à 150 ou 180 moles d'oxyde d'éthylène, de l'alcool stéarylique et du méthylène bis(4-cyclohexyl-isocyanate) (SMDI), à 15% en poids dans une matrice de maltodextrine (4%) et d'eau (81%).

Parmi les polyéthers polyuréthanes (b), on peut citer le produit vendu par la société ROHM & HAAS sous la dénomination commerciale :
ACULYN 44, qui est un polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%).

On peut également utiliser, en tant que polyuréthanne associatif, le Viscophobe DB 1000 (Union Carbide).

Comme autres polymères associatifs utilisables, on peut notamment citer les copolymères acide acrylique (Meth) / acrylate d'alkyle C10-C30 comme le Pemulen TR1 de Goodrich.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄, des cétones aliphatiques ou aromatiques, des esters d'acides à chaînes courtes en C₁ à C₈ ou longues et C₉ à C₂₀ et d'alcools à chaînes courtes C₁ à C₈ ou longues C₉ à C₂₀, le pentane, l'heptane, les polyols, les éthers de polyol et l'isodécane.

Parmi les alcools, on peut citer l'éthanol, l'isopropanol. L'éthanol est particulièrement préféré.

Le rapport entre la concentration en poids des polyuréthanes de haut poids moléculaire et la concentration en poids des épaississants, dans les compositions conformes à l'invention, est avantageusement compris entre 0,01 et 100, plus avantageusement entre 0,05 et 20, et plus avantageusement encore entre 0,1 et 10.

La composition de l'invention peut également contenir au moins un additif choisi parmi les tensioactifs anioniques, cationiques, non ioniques ou amphotères, les parfums, les filtres, les conservateurs, les protéines, les vitamines, les provitamines, les polymères autres que ceux de l'invention, les huiles végétales, minérales ou synthétiques, les polyols comme les glycols ou la glycérine, les silicones, les alcools gras et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions conformes à l'inventions peuvent être appliquées sur la peau, les ongles, les lèvres, les cheveux, les sourcils et les cils.

Les compositions conformes à l'invention sont particulièrement adaptées pour des cheveux secs ou humides, en tant que produits de coiffage.

L'invention va être plus complètement illustrée à l'aide des exemples non limitatifs suivants.

Tous les pourcentages sont des pourcentages relatifs en poids par rapport au poids total de la composition et m.a. signifie matière active.

### EXEMPLE :

### Nous préparons un gel de coiffage selon l'invention :

a. Avalure UR 450 (Noveon).....7% m.a.
b. Jaguar HP105 (Rhodia) .....1% m.a.
c. Q2-5220 (Dow Corning).....1% m.a.
d. eau.....qsp 100% m.a.

Le gel de coiffage selon l'invention a une longue tenue dans le temps. La coiffure réalisée avec le gel selon l'invention reste intacte après être plongée pendant 30 minutes dans un bac d'eau douce.

## Revendications

1. Composition cosmétique se présentant sous la forme d'un gel et comprenant, dans un milieu cosmétiquement acceptable, au moins un polyuréthane de poids moléculaire moyen en nombre compris entre 400 000 et 5 000 000 g/mol et au moins un épaississant.

2. Composition de traitement cosmétique des matières kératiniques selon la revendication 1, **caractérisé en ce que** le polyuréthane présente une masse moléculaire comprise entre environ 400 000 et 3 000 000, de préférence entre 1 000 000 et 2 500 000.

3. Composition de traitement cosmétique des matières kératiniques selon la revendication 1, **caractérisé en ce que** le polyuréthane est non associatif.

4. Composition de traitement cosmétique des matières kératiniques selon l'une quelconque des revendications précédentes, **caractérisée en ce que** le polyuréthane comprend un motif répétitif de base répondant à la formule générale (I) :
-O-B-O-CO-NH-R-NH-CO- (I)
dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes hydrocarbonés aliphatiques en C₁ à C₂₀, cycloaliphatiques en C₃ à C₂₀ et aromatiques en C₆ à C₂₀,
ou leurs combinaisons, ces groupes étant substitués ou non.

5. Composition de traitement cosmétique des matières kératiniques selon l'une quelconque des revendications 3 à 7, **caractérisée en ce que** le polyuréthane est présent en une quantité de 0,05 à 40 % en poids, de préférence de 0,10 à 20 % en poids par rapport au poids total de la composition.

6. Composition selon l'une quelconque des revendications précédentes, **caractérisée par le fait que** l'agent épaississant est choisi parmi les polymères naturels ou naturels modifiés (i), les copolymères réticulés d'acide acrylique et/ou méthacrylique (ii), les polyacrylamides épaississants réticulés (iii) et les polymères associatifs comportant au moins un motif hydrophile et au moins une chaîne grasse (iv) .

7. Composition selon la revendication 6, **caractérisée par le fait que** le polymère naturel ou naturel modifié (i) est choisi parmi les gommes de xanthane, de cellulose, de scléroglucane, de gellane, de rhamsan, les alginates, la gomme de karaya, la farine de caroube, les gommes de guar et leurs dérivés.

8. Composition selon la revendication 6, **caractérisée par le fait que** les copolymères d'acide acrylique et/ou méthacrylique réticulés (ii) sont choisis parmi les copolymères acide acrylique/acrylate d'éthyle et les polymères carboxyvinyliques.

9. Composition selon la revendication 6, **caractérisée par le fait que** les polyacrylamides épaississants réticulés (iii) sont choisis parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium réticulés,
- les copolymères d'acrylamide et d'acide 2-acrylamido 2-méthyl propanesulfonique partiellement ou totalement neutralisés réticulés.

10. Composition selon la revendication 6, **caractérisée par le fait que** les polymères associatifs (iv) sont des polyuréthannes associatifs.

11. Composition selon l'une quelconque des revendications précédentes, **caractérisée par le fait que** la composition comprend, en pourcentage en poids de la composition, de 0,01 à 20 % d'épaississant, et de préférence, de 0,05 à 10 % .

12. Composition selon l'une quelconque des revendications précédentes, **caractérisée par le fait que** le rapport entre la concentration en poids des polyuréthanes de haut poids moléculaire et la concentration en poids des épaississants, dans les compositions conformes à l'invention, est compris entre 0,01 et 100, plus avantageusement entre 0,05 et 20, et plus avantageusement encore entre 0,1 et 10.

13. Composition selon l'une quelconque des revendications précédentes, **caractérisée par le fait qu'**elle contient au moins un additif choisi parmi les tensioactifs anioniques, cationiques, non ioniques ou amphotères, les parfums, les filtres, les conservateurs, les protéines, les vitamines, les provitamines, les polymères autres que ceux de l'invention, les huiles végétales, minérales ou synthétiques, les polyols comme les glycols ou la glycérine, les silicones, les alcools gras et tout autre additif classiquement utilisé dans les compositions cosmétiques.

14. Procédé cosmétique capillaire, en particulier un procédé de fixation et/ou de maintien de la coiffure mettant en oeuvre une composition selon l'une quelconque des revendications 1 à 13.

15. Utilisation d'une composition selon l'une quelconque des revendications 1 à 13 dans une formulation cosmétique capillaire, destinée notamment au maintien et/ou à la mise en forme de la coiffure.
